# EUROPEAN PATENT SPECIFICATION

(11) **EP 4 452 092 B1**
(45) Date of publication and mention of the grant of the patent: **13.05.2026**
(21) Application number: 22786465.9
(22) Date of filing: 22.09.2022
(51) Int. Cl.: A61B 17/02, A61B 34/20, A61B 34/30, A61B 90/00

(54) **BILATERAL ROBOTIC SPINAL ENDOSCOPY**
BILATERALE ROBOTISCHE WIRBELSÄULENENDOSKOPIE
ENDOSCOPIE SPINALE ROBOTIQUE BILATÉRALE

(30) Priority: 20.12.2021 US 202163291460 P
(43) Date of publication of application: 30.10.2024
(73) Proprietor: LEM SURGICAL AG, 3018 Bern (CH)
(72) Inventor: BAR, Yossi, 3074 Muri bei Bern (CH)
(74) Representative: HGF
(86) International application number: PCT/IB2022/058982
(87) International publication number: WO 2023/118985

(56) References cited:
- WO-A1-2021/050767
- WO-A1-2021/250580
- CN-A- 109 549 706
- US-A1- 2021 361 357

## Description

### FIELD OF THE INVENTION

The invention relates to systems and method for robotically controlled and coordinated spinal endoscopy procedures. In particular, the invention relates to robotic systems comprising multiple robotic elements, such as robotic arms, end effectors, surgical instruments, cameras, imaging devices, tracking devices, sensors or other devices useful for robotic spinal endoscopy. The invention also relates to robotic systems wherein the placement and movement of the robotic elements are controlled and coordinated by a single control unit, and wherein all of the robotic elements are based on a single rigid mobile chassis and, thus, are robotically coordinated at a single origin point. Specifically, multiple robotic elements may be attached to, and controlled by, a single control unit and may be used in a coordinated fashion to deploy and/or relate to surgical instruments, trackers, cameras, and other surgical tools as part of a robotic spinal endoscopy procedure. More particularly, in the context of robotic spinal surgery, multiple end effectors may be deployed on multiple robotic arms and controlled by a single control unit and may be used in a centrally coordinated fashion to perform a robotic surgical procedure, with the relative movements of each robotic element being coordinated by the central control unit. Most particularly, in the context of minimally invasive spinal endoscopy procedures, the safety, efficiency and precision of a bilateral approach to the procedure may be enabled by surgical tools, sensors and cameras being deployed on multiple robotic arms that are controlled in a synchronized and coordinated fashion to perform a safe and efficient surgical procedure.

### BACKGROUND OF THE INVENTION

Robotic surgery is well known in the art, as is the application of robotic techniques to spinal surgery procedures. Many robotic surgery systems, such as the da Vinci robotic surgery system from Intuitive Surgical, are teleoperated. Multi-arm robotic surgical systems are available in the field, for example those provided by Cambridge Medical Robotics, but these known systems are often also teleoperated and are all comprised of single arms deployed separately on separate carts or chassis with some level of coordination provided by a remotely-positioned control unit. Systems comprising multiple arms on multiple carts have significant drawbacks regarding integration into surgical workflow, along with an undesirably large footprint in the operating room. Also, the control of teleoperated units by a remotely-positioned control unit does not provide the level of control required for a full range of surgical procedures, particularly in the case of spinal surgery. Accuracy will inevitably be inferior to a system where all robotic arms are fixed to, and coordinated by, a single chassis comprising a single control unit. This is particularly important in the context of spinal endoscopy, which requires an even greater level of precision and control than other spinal surgical procedures due to the need to safely position ports/trocars and keep them in position throughout the duration of the surgical procedure.

Performance of a full range of spinal surgery procedures, including spinal endoscopy, requires robotically coordinated navigation which is not available today. A typical procedure may require the maneuvering of one or more end effectors deployed by robotic arms, the deployment of other instruments, placement of multiple passive or active markers on bone and/or on soft tissue or on instruments or robotic arms, and one or more robotically controlled and maneuvered cameras that can be placed at varying distances and angulations from the surgical field, and one or more end effectors deployed by robotic arms. Such a mobile bi-lateral multi-arm/multi-camera system mounted on, and controlled by, one cart, is not available in the current state of the art. There is a strong and long-felt need for such a system as it will enable the performance of safe and precise spinal endoscopy procedures with robotically coordinated control and navigation at a level of accuracy not currently possible. A robotically coordinated bilateral approach to spinal endoscopy is likely even more preferred.

The need for a centrally coordinated multi-faceted approach is felt strongly in spinal endoscopy procedures. The endoscopic approach in spinal surgery is the most minimally invasive approach and therefore is attractive to patients, surgeons and hospitals. Spinal endoscopy, despite being attractive, is not performed very often due to the difficulties and risks inherent in the procedure at current state of the art. In most applications, it is very difficult to place and maintain the ports/trocars/working channels in a safe location, with safety being defined as not impacting nervous structures.

One current option for enhancing safety is the use of x-rays, but x-rays cannot visualize delicate nerve roots and there is a concern about excessive exposure to radiation. Accordingly, surgeons attempting spinal endoscopy procedures will very often navigate according to the position of the bony anatomy and guessing where the nerve structures are. This poses an unacceptable risk of catastrophic damage. Moreover, usually the angles through which endoscopic ports are inserted in spinal endoscopy are relatively wide (over 30deg in the axial plane) which intrinsically makes the x-ray image very difficult to understand and interpret due to its oblique nature.

Another problem with using x-ray guidance for spinal endoscopy procedures is the imposition of large amounts of harmful radiation on the patient and the surgeon. If multiple x-rays are required to confirm the position of the working channels with respect to the bony anatomy during the course of the surgical procedure, then the harmful effects of radiation are only multiplied.

Difficulty controlling and maintaining the safe positioning of the working channels during surgery is only made worse by the fact that their position and trajectory may actually need to be intentionally adjusted multiple times during the surgical procedure, such as when access to a different portion of the spinal anatomy is required. Each of these adjustments brings a safety risk, may require more x-rays (and, thus, more radiation exposure), and is also time consuming and difficult for the surgeon to perform properly.

Bilateral approaches to surgical endoscopy have been known for some time in the surgical arts, although they are similarly practiced relatively rarely. A bilateral approach can allow, for example, access to multiple facets of the surgical field or region of interest. This approach can also allow, for example, grasping with one end effector and manipulation with another end effector. However, in the context of spinal endoscopy, a bilateral approach also carries with it essentially double the risks of a single port spinal endoscopy approach. Each of the working channels may inadvertently move during surgery or may need to be intentionally repositioned. Multiple x-rays may thus be required. Even then, the risk of injury to nervous structures will not be completely eliminated.

A system that robotically synchronizes the placement and movement of the working channels/ports/trocars in a bilateral approach would ameliorate this concern to a great extent because the ports would be held and placed by robotic arms whose movements are synchronized with each other, thus reducing the risk of harm. The robotic system would place and hold the ports with the guidance of one or more cameras and markers, all of which would be visualized and coordinated by a single-chassis base with a central control unit. Such a system is provided in the context of the present invention.

WO 2021/250580 describes a system for endoscopic surgery, comprising a first robotic arm defining the pose of an endoscopic camera, a second robotic arm defining the pose of a surgical end effector, and an irrigation nozzle configured to inject fluid under a predetermined pressure into a preselected tissue region, to generate a cavity at a surgical site.

US 2021/361357 describes a surgical implant planning computer for intra-operative CT workflow, pre-operative CT imaging workflow, and fluoroscopic imaging workflow. A network interface is connectable to a CT image scanner and a robot surgical platform having a robot base coupled to a robot arm that is movable by motors.

CN 109549706 describes a surgical operation auxiliary system and an application method thereof. The surgical operation auxiliary system comprises a host computer, a mechanical arm, input equipment, displaying equipment, a guiding device and the like.

WO 2021/050767 describes systems and methods for accessing and modulating tissue (for example, systems and methods for accessing and ablating nerves or other tissue within or surrounding a vertebral body to treat chronic lower back pain).

### SUMMARY OF THE INVENTION

Aspects of the invention are as set out in the appended claims.

Provided herein is a mobile bi-lateral robotically controlled surgical system. Specifically, the inventive system is a centrally coordinated and synchronized robotic system for spinal robotic endoscopy procedures, optionally for bilateral spinal robotic endoscopy procedures. The system comprises multiple robotic arms that each can hold, place and/or manipulate at least one end effector, port, camera/sensor or navigation/tracking element for use in a spinal endoscopy procedure. The ports may take the form of working channels or trocars for providing access to the surgical field for end effectors. The end effectors may include any surgical tools useful for performing spinal endoscopy procedures. The cameras/sensors and navigation/tracking elements (including, but not limited to, force/torque sensing, CT, MRI etc.) are for providing guidance for the movement of the robotic arms and deployment of the ports, end effectors and tools.

The invention comprises multiple robotic arms which access and visualise the surgical field in an automatic and safe way because they are robotically synchronized. In one embodiment, there may be three robotic arms, two of which place, guide and/or hold ports and one holding a navigation camera. In such an embodiment, the arms holding the ports may, after the ports have been placed, bring and manipulate other end effectors in the surgical field. In such an embodiment, the first arm may optionally position a port and then control the use of, for example, a drilling tool. The second arm may optionally position a second port and then control the use of, for example, a grasping tool. A third arm may optionally hold a camera that provides an image of the process from an optimal distance and angulation. The camera is able to operate from optimal distance and angulation because it is sized appropriately and its deployment on an appropriately sized and positioned robotic arm. Optionally, the robotic arms may also hold additional imaging or navigation cameras to provide redundancy and diversity of information. Also optionally, the robotic arms and/or the ports or end effectors may have active or passive markers placed on them that may assist the robotic system in positioning the robotic arms, the ports and/or the end effectors.

In one embodiment, the synchronized movement of the robotic arms is enabled by the interaction of the navigation cameras with active or passive markers that are placed at the beginning of the procedure on portions of the patient's anatomy. The movement of the robotic arms is synchronized by a central control unit from a single base that knows where the arms are based upon the navigation information provided by the various markers and the one or more cameras.

Accordingly, in various embodiments of the inventive system, passive or active markers may be used to assist in navigation during a spinal endoscopy procedure that may employ a bilateral approach. These procedures may require the placement of multiple passive or active markers on the patient's anatomy or on the robotic arms, end effectors or ports. In particular embodiments, miniature markers may be preferred. Portions of the patient's anatomy may be relatively small and so to place multiple markers on different anatomical portions, it may be advantageous to use relatively small markers (1 cm or less in size). When using small markers, it may be advantageous to have the one or more cameras be deployed quite close to the surgical field, for example at a distance of 50cm or less from the surgical field, and also at an advantageous angulation relative to the surgical field so that the marker(s) can be visualized. It may also be advantageous to place smaller markers on the robotic arms, end effectors or ports so that they do not obscure each other or aspects of the surgical field. This arrangement can then provide appropriate navigation information to the central control unit and provide for coordinated movement of the robotic arms in their placement and maintenance of the surgical ports and use of the end effectors.

The inventive approach allows for synchronized operation of the multiple robotic arms, and in many embodiments three arms may be chosen. One robotic arm may hold, position and maintain one endoscopic port. A second robotic arm may hold, position and maintain a second endoscopic port which may also include an endoscopic camera. A third robotic arm may position and hold a navigation camera. The robotic arms responsible for placing the endoscopic ports may also optionally deploy end effectors to the surgical field through the endoscopic ports.

In one embodiment of the present invention, two robotic arms may simultaneously guide two ports in a bilateral approach to both sides of the patient and, thus, to both sides of the relevant surgical field. The bi-lateral ports may be conventional endoscopy ports of e.g. 5-10mm in diameter. In an alternative related embodiment, three robotic arms may simultaneously guide three ports in a multilateral approach to multiple aspects of the surgical field. While this is theoretically enabled by the centralized control of the present robotic system, the present inventor believes that it has limited applications in surgery. In alternative related embodiments, the at least two robotic arms may sequentially guide the at least two ports rather than simultaneously guiding them.

In various embodiments of the present invention, an additional robotic arm mechanically connected to the same chassis of as the at least two robotic arms that guide the endoscopy ports may position and hold a navigation camera. The navigation camera may view the entire surgical field, and in particular the two ports that are held by the two other robotic arms in most bilateral spinal endoscopy procedures.

In some embodiments of the present invention, guide wires that confirm accurate placement of the ports may be used. In one example, a 1-3mm guide wire is used and can be inserted into a port after it has been placed or preferably before. In many spinal endoscopic procedures, the port/canula is inserted just below the delicate nerve root with the intention of minimal contact with it. It will be advantageous to place a thin instrument/wire in said trajectory before inserting the wider port all the way and close to the nerve root and after proper position verification only than inserting the wider diameter port. The guide wire is significantly smaller in diameter than the port and, thus, carries much less risk to the patient. More over the guide wire is thin and rigid therefore inhibits less resistance with the soft tissue, meaning less deviations and more accurate placement. The guide wires can be used to confirm proper placement of the endoscopic ports with higher accuracy and less risky. Moreover, the guide wires may be equipped with electrophysiological sensor (passive or active) which can provide another layer of safety. Another verification metho can be to insert the wire through the canula from one side and from the other side to insert though he canula an endoscopic camera. This camera can serve for visual verification of the successful arrival of the first wire or the current insertion and/or manipulation of a surgical tool through the first arm. The endoscopic camera can of course be inserted from both sides, together or separately.

In one embodiment, a method of using the robotic system of the present invention is provided in the context of a robotic bilateral spinal endoscopy procedure. In this inventive method, after the robotic arms carrying the ports have reached the appropriate position and trajectory as determined by pre-operative planning and navigation provided by a camera on a third robotic arm (with the possible assistance of miniature markers), the ports are inserted into the patient's soft tissue and insertion is stopped at a safe position with respect to the spinal anatomy (and in particular with respect to delicate nervous structures). Before advancing the ports any further, the surgeon may insert guide wires (e.g.,1-3mm wide) into the ports and advance them further through the patient's soft tissue, eventually puncturing the disc annulus on either side of the surgical field. In one example of usage of this technique if the trajectory of insertion of both ports and guide wires has been planned and executed correctly, the guide wires will come into contact with each other in the designated anatomical field (i.e., disc space). The robotic system can determine that the guide wires have come into contact with each other by electrical conductivity for example. This will, in turn, allow the robotic system and with that also to the surgeon to determine that it is safe to advance the ports further into the surgical field to the point where relevant tools or end effectors can be advanced through the ports to perform the surgical procedure.

In various embodiments of the present invention, the guide wires can be deployed manually by the surgeon or completely automatically by the surgical robot.

The inventive embodiments take advantage of multiple feedback loops to ensure precision and safety in the performance of the bilateral robotic spinal endoscopy procedure. The movement of the robotic arms is robotically synchronized to the greatest possible level of precision because the arms are all co-mounted on a single chassis that has a central control unit. Robotic navigation is provided by one or more cameras that are deployed by one or more robotic arms that are also co-mounted on the same single chassis and are also controlled by the same central control unit. Thanks to the special system morphology, the distance between the robotic camera/sensor and the navigated/tracked markers is minimal. Due to the close vicinity of the camera/sensor, the tracked markers are extremely accurate since they are small and upholds minimal movement due to its size. Also, a closed loop of electrical conductivity allows the robot and/or the user to confirm appropriate placement of the endoscopy ports with minimal risk so that the surgeon knows that it is safe to proceed with the other phases of the spinal endoscopy procedure.

All of these needs and elements benefit tremendously from the central coordination and synchronized control of the inventive single-cart, multi-arm, non-teleoperated robotic system. Based on the placement of appropriately sized markers and the placement of navigation cameras at an appropriate distance and orientation to the target anatomy and the markers, movement of the robotic arms carrying end effectors and cameras can be coordinated to provide for a safe and precise robotic spinal bi-lateral endoscopy procedure.

### BRIEF DESCRIPTION OF THE DRAWINGS

- Figures 1 shows a bilateral approach to a spinal endoscopy procedure using the inventive system described herein.
- Figure 2 is an alternative close-up view of a bilateral approach to an endoscopic spinal surgical procedure using the system of the present invention.

### DETAILED DESCRIPTION OF THE INVENTION

With reference now to the figures and several representative embodiments of the invention, the following detailed description is provided.

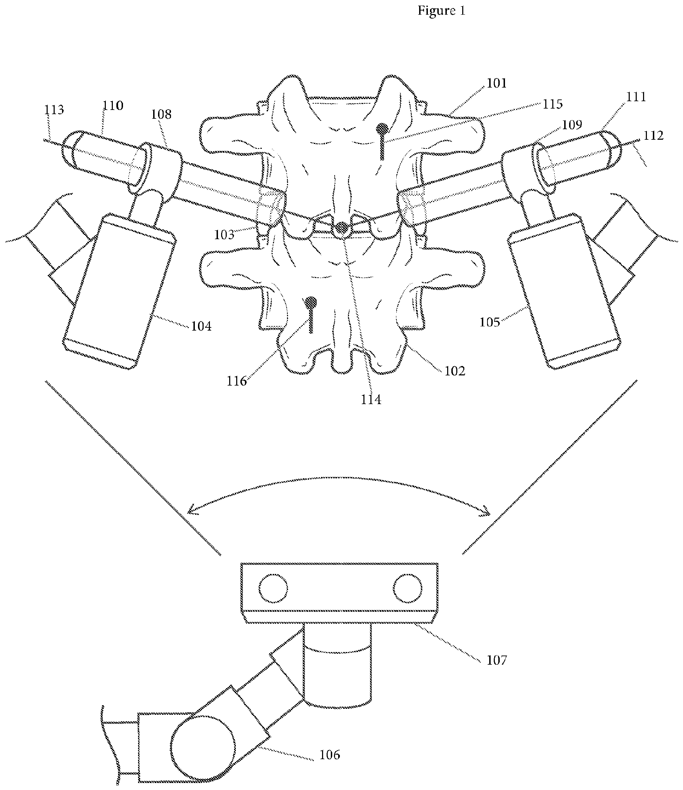

In a working example of the invention exemplified by Figure 1, a bilateral, minimally invasive, endoscopic approach to spinal surgery is shown. The surgical procedure is being performed on or near a portion of the spinal bony anatomy exemplified by an upper vertebra portion 101 and a lower vertebra portion 102. In particular, the surgical procedure is being performed in or near the disc space 103, which one of skill in the art will realize is directly adjacent to the delicate nerve roots.

The surgeon may optionally wish to place active or passive markers 115, 116 on the bony anatomy at or near in time to the beginning of the surgical procedure. The placement of these markers 115, 116 can assist a centrally coordinated robotic navigation system to position robotic arms holding instruments and cameras at or adjacent to the patient's anatomy of interest. The markers may optionally be relatively small (1 cm or less in size) so that they don't interfere with the view of the surgical field obtained by the surgeon or the robotic navigation system.

A centrally coordinated robotic navigation system, such as a system provided by the present applicants, is shown performing the surgical procedure in Figure 1. The exemplary robotic system shown in Figure 1 has three robotic arms 104, 105, 106. The robotic arms are mounted on a single, central chassis and their motion is coordinated by a central control unit disposed in the central chassis. Robotic arms 104 and 105 each deploy port holders 108 and 109 and robotic arm 106 holds a robotic navigation camera 107. The robotic navigation camera 107 is positioned to advantageously view the surgical field, including the markers 115, 116, and thus can assist in guiding the robotic arms 104, 105 to the relevant portion of the surgical field. The robotic navigation camera 107 can be held within one meter or less of the patient's anatomy of interest without interfering with the surgeon's view and while appropriately visualizing the anatomy and the surgical markers.

The system of the present invention as exemplified in Figure 1 includes a first port 110 and a second port 111 that are positioned and held by the port holders 108 and 109 respectively that are, in turn, held by robotic arms 104 and 105 respectively. The bi-lateral ports may be conventional endoscopy ports of 5-10mm in diameter. The ports 110 and 111 may be advanced near to the area of interest, for example to a position just outside the disc space 103. A first conductive wire 113 and a second conductive wire 112 may then be advanced through the ports 110 and 111 respectively, until they intersect at a point 114 in the anatomy of interest, for example in the disc space. The conductive wires 113, 112 may be advantageously e.g. 1-3mm in size so as to advance through the delicate anatomical region without undue risk and relatively with low soft tissue resistance, hence more accurately. The robotic system may optionally receive a signal when the conductive wires 113 and 112 have reached the intersection point 114, such as by electrical conductivity being established between the wires. Once the surgeon and/or the system controller knows/signaled that the wires have reached the intersection point 114, the ports 110 and 111 may then be advanced along the same path as the conductive wires 113 and 112 with confidence that a safe path has been found that avoids the delicate nerve roots in the adjacent anatomy.

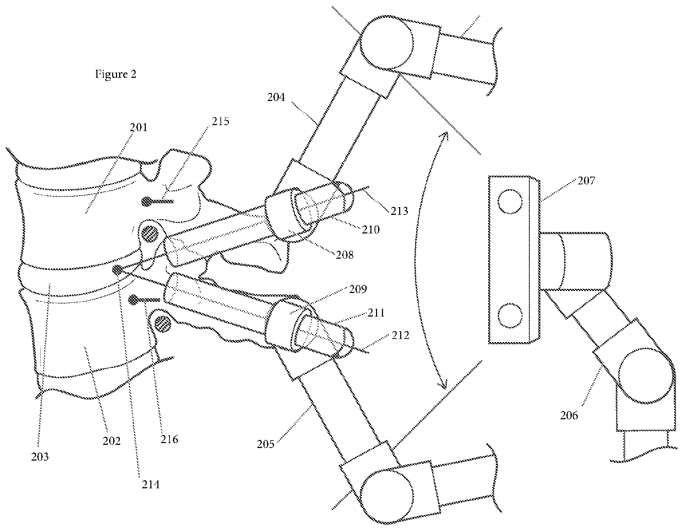

In a second working example of the invention exemplified by Figure 2, an alternate lateral view of a bilateral, minimally invasive, endoscopic approach to spinal surgery is shown. The surgical procedure is being performed on or near a portion of the spinal bony anatomy exemplified by an upper vertebra portion 201 and a lower vertebra portion 202. In particular, the surgical procedure is being performed in or near the disc space 203, which one of skill in the art will realize is directly adjacent to the delicate nerve roots.

The surgeon may optionally wish to place active or passive markers 215, 216 on the bony anatomy at or near in time to the beginning of the surgical procedure. The placement of these markers 215, 216 can assist a centrally coordinated robotic navigation system to position robotic arms holding instruments and cameras at or adjacent to the patient's anatomy of interest.

A centrally coordinated robotic navigation system, such as a system provided by the present applicants, is shown performing the surgical procedure in Figure 2. The exemplary robotic system shown in Figure 2 has three robotic arms 204, 205, 206. The robotic arms are mounted on a single, central chassis and their motion is coordinated by a central control unit disposed in the central chassis. Robotic arms 204 and 205 each deploy port holders 208 and 209 and robotic arm 206 holds a robotic navigation camera 207. The robotic navigation camera 207 is positioned to advantageously view the surgical field, including the markers 215, 216, and thus can assist in guiding the robotic arms 204, 205 to the relevant portion of the surgical field.

The system of the present invention as exemplified in Figure 2 includes a first port 210 and a second port 211 that are positioned and held by the port holders 208 and 209 respectively that are, in turn, held by robotic arms 204 and 205 respectively. The ports 210 and 211 may be advanced near to the area of interest, for example to a position just outside the disc space 203. A first conductive wire 213 and a second conductive wire 212 may then be advanced through the ports 210 and 211 respectively, until they intersect at a point 214 in the anatomy of interest, for example in the disc space. The robotic system may optionally receive a signal when the conductive wires 213 and 212 have reached the intersection point 214, such as by electrical conductivity being established between the wires. Once the surgeon knows that the wires have reached the intersection point 214, the ports 210 and 211 may then be advanced along the same path as the conductive wires 213 and 212 with confidence that a safe path has been found that avoids the delicate nerve roots in the adjacent anatomy.

The inventive embodiments take advantage of multiple feedback loops to ensure precision, efficiency and safety in the performance of the bilateral robotic spinal endoscopy procedure. The movement of the robotic arms is robotically synchronized to the greatest possible level of precision because the arms are all co-mounted on a single chassis that has a central control unit. Robotic navigation is provided by one or more cameras that are deployed by one or more robotic arms that are also co-mounted on the same single chassis and are also controlled by the same central control unit. Also, a closed loop of electrical conductivity allows the robot and/or the user to confirm appropriate placement of the endoscopy ports so that the surgeon knows that it is safe to proceed with the other phases of the spinal endoscopy procedure. All of these feedback loops also provide great efficiency in the performance of the robotic bilateral endoscopy procedure because when the surgeon is assured of safety and precision, they can also move through the procedure with confidence, and thus, relative speed.

In particular, the inventive embodiment demonstrates the benefits of a bilateral approach to robotically coordinated spinal endoscopy. The inventive system minimizes or removes altogether many of the risks associated with conventional spinal endoscopy. The system allows for much more precise placement of working channels, thus minimizing risk to nervous system structures. Also, the use of markers that are seen by the robotic system allows for the placement of trocars and movement of instruments with far less risk to the patient while still avoiding excessive radiation exposure to the patient.

All of these needs and elements benefit tremendously from the central coordination and synchronized control of the inventive single-cart, multi-arm, non-teleoperated robotic system. Based on the placement of appropriately sized markers and the placement of navigation cameras at an appropriate distance and orientation to the target anatomy and the markers, movement of the robotic arms carrying end effectors and cameras can be coordinated to provide for a safe and precise robotic spinal bi-lateral endoscopy procedure. The centrally coordinated robotic navigation system provided by the applicants as part of the inventive system is premised on the notion of mounting multiple robotic arms on a single, central chassis, wherein the central chassis also comprises a central control unit. The central control unit coordinates the movements of 3 or more robotic arms that deploy surgical instruments and navigation cameras and are guided by navigation information provided by the navigation cameras and active or passive markers. In the present invention, the central control unit coordinates the movement of two robotic arms deploying port holders with additional instrumentation and/or endoscopic cameras in it and one or more robotic arm holding a navigation camera. The system is then able to guide surgical ports, held by the port holders, to spinal anatomy of interest through navigation information provided by the navigation camera and optionally placed active or passive markers.

One of skill in the art will realize that several variations on the disclosed embodiments are possible while staying within the bounds of the current invention. Solely by way of example, different variations in the number of navigation cameras, robotic arms, markers and end effectors can be used without departing from the invention. As another example, markers of varying sizes can be used. As yet another example, numerous variations of surgical tools and surgical approaches to bilateral spinal endoscopy can be employed without departing from the invention described herein. The embodiments provided are representative in nature.

## Claims

1. A system for robotically coordinated spinal endoscopy at a patient's target anatomy in a surgical field, said system comprising:
a robotic system comprising at least three robotic arms (104, 105, 106) mounted on a single chassis housing a central control unit;
a first robotic arm (104) of the at least three robotic arms holding a device providing a first minimally invasive working channel
configured to carry a first thin and rigid wire (113) to the surgical field from a first side of a patient's body;
a second robotic arm (105) of the at least three robotic arms holding a device providing a second minimally invasive working channel configured to carry a second thin and rigid wire (112) to the surgical field from a second side of the patient's body;
a third robotic arm (105) of the at least three robotic arms holding an imaging device (107) or sensor; and
at least one marker element (115, 116) attached to an aspect of the patient's bony anatomy in the surgical field; **characterized in that**
wherein the central control unit is configured to position the first and second minimally invasive working channels to locate the first and second thin and rigid wires (113, 112) carried by the first and second minimally invasive working channels at the target anatomy and to detect contact between the wires to confirm proper placement of the minimally invasive working channels;
wherein the first minimally invasive working channel and the second minimally invasive working channel are configured to provide safe access to the surgical field without the need for x-ray radiation and without risking damage to delicate nervous system structures.

2. The system of claim 1, wherein the robotic system is mobile.

3. The system of claim 1, wherein the imaging device (107) or sensor is a surgical navigation camera.

4. The system of claim 1, wherein at least two marker elements (115, 116) are attached to two adjacent aspects of the patient's bony anatomy in the surgical field.

5. The system of any of the preceding claims, wherein the movement of the robotic arms (104, 105, 106) is robotically coordinated by the central control unit relative to a single origin point.

6. The system of claim 3, wherein the first robotic arm (104) and the second robotic arm (105) are positioned by the central control unit in position with respect to the patient's bony anatomy with the assistance of the surgical navigation camera (107).

7. The system of any of the preceding claims, wherein the central control unit is further configured to detect electrical contact between the first thin rigid wire (113) and the second thin rigid wire (112).

## Patentansprüche

1. System für robotisch koordinierte Wirbelsäulenendoskopie an der Zielanatomie eines Patienten in einem Operationsfeld, wobei das System Folgendes umfasst:
ein Robotersystem, das mindestens drei Roboterarme (104, 105, 106) umfasst, die an einem einzigen Gestell montiert sind, das eine zentrale Steuereinheit aufnimmt;
wobei ein erster Roboterarm (104) der mindestens drei Roboterarme eine Vorrichtung hält, die einen ersten minimalinvasiven Arbeitskanal bereitstellt,
der dazu konfiguriert ist, einen ersten dünnen und starren Draht (113) von einer ersten Seite des Körpers eines Patienten zu dem Operationsfeld zu führen;
ein zweiter Roboterarm (105) der mindestens drei Roboterarme eine Vorrichtung hält, die einen zweiten minimalinvasiven Arbeitskanal bereitstellt, der dazu konfiguriert ist, einen zweiten dünnen und starren Draht (112) von einer zweiten Seite des Körpers des Patienten zu dem Operationsfeld zu führen;
ein dritter Roboterarm (105) der mindestens drei Roboterarme eine Bildgebungsvorrichtung (107) oder einen Sensor hält; und
mindestens ein Markierungselement (115, 116), das an einem Aspekt der Knochenanatomie des Patienten in dem Operationsfeld befestigt ist; **dadurch gekennzeichnet, dass**
wobei die zentrale Steuereinheit dazu konfiguriert ist, den ersten und den zweiten minimalinvasiven Arbeitskanal zu positionieren, um den ersten und den zweiten dünnen und starren Draht (113, 112), die durch den ersten und den zweiten minimalinvasiven Arbeitskanal geführt werden, an der Zielanatomie anzubringen und einen Kontakt zwischen den Drähten zu erkennen, um die ordnungsgemäße Platzierung der minimalinvasiven Arbeitskanäle zu bestätigen;
wobei der erste minimalinvasive Arbeitskanal und der zweite minimalinvasive Arbeitskanal dazu konfiguriert sind, einen sicheren Zugang zu dem Operationsfeld ohne die Notwendigkeit einer Röntgenstrahlung und ohne das Risiko einer Schädigung empfindlicher Strukturen des Nervensystems bereitzustellen.

2. System nach Anspruch 1, wobei das Robotersystem mobil ist.

3. System nach Anspruch 1, wobei die Bildgebungsvorrichtung (107) oder der Sensor eine chirurgische Navigationskamera ist.

4. System nach Anspruch 1, wobei mindestens zwei Markierungselemente (115, 116) an zwei benachbarten Aspekten der Knochenanatomie des Patienten in dem Operationsfeld befestigt sind.

5. System nach einem der vorhergehenden Ansprüche, wobei die Bewegung der Roboterarme (104, 105, 106) durch die zentrale Steuereinheit in Bezug auf einen einzigen Ausgangspunkt robotergestützt koordiniert wird.

6. System nach Anspruch 3, wobei der erste Roboterarm (104) und der zweite Roboterarm (105) durch die zentrale Steuereinheit in Bezug auf die Knochenanatomie des Patienten mit Hilfe der chirurgischen Navigationskamera (107) in Position positioniert werden.

7. System nach einem der vorhergehenden Ansprüche, wobei die zentrale Steuereinheit ferner dazu konfiguriert ist, einen elektrischen Kontakt zwischen dem ersten dünnen starren Draht (113) und dem zweiten dünnen starren Draht (112) zu erkennen.

## Revendications

1. Système d'endoscopie spinale coordonnée par robot au niveau de l'anatomie cible d'un patient dans un champ opératoire, ledit système comprenant :
un système robotique comprenant au moins trois bras robotiques (104, 105, 106) montés sur un seul châssis logeant une unité de commande centrale ;
un premier bras robotique (104) parmi lesdits au moins trois bras robotiques tenant un dispositif fournissant un premier canal de travail minimalement invasif conçu pour transporter un premier fil mince et rigide (113) vers le champ opératoire à partir d'un premier côté du corps d'un patient ;
un deuxième bras robotique (105) parmi lesdits au moins trois bras robotiques tenant un dispositif fournissant un second canal de travail minimalement invasif conçu pour transporter un second fil mince et rigide (112) vers le champ opératoire à partir d'un second côté du corps du patient ;
un troisième bras robotique (105) parmi lesdits au moins trois bras robotiques tenant un dispositif d'imagerie (107) ou un capteur ; et
au moins un élément marqueur (115, 116) fixé à un aspect de l'anatomie osseuse du patient dans le champ opératoire ;
**caractérisé en ce que**
l'unité de commande centrale est configurée pour positionner les premier et second canaux de travail minimalement invasifs de manière à localiser les premier et second fils minces et rigides (113, 112) portés par les premier et second canaux de travail minimalement invasifs au niveau de l'anatomie cible et pour détecter un contact entre les fils de manière à confirmer le placement correct des canaux de travail minimalement invasifs ;
le premier canal de travail minimalement invasif et le second canal de travail minimalement invasif sont conçus pour fournir un accès sûr au champ opératoire sans nécessiter de rayonnement de rayons X et sans risquer d'endommager les structures délicates du système nerveux.

2. Système selon la revendication 1, ledit système robotique étant mobile.

3. Système selon la revendication 1, ledit dispositif d'imagerie (107) ou ledit capteur étant une caméra de navigation chirurgicale.

4. Système selon la revendication 1, au moins deux éléments marqueurs (115, 116) étant fixés à deux aspects adjacents de l'anatomie osseuse du patient dans le champ opératoire.

5. Système selon l'une quelconque des revendications précédentes, ledit mouvement des bras robotiques (104, 105, 106) étant coordonné par robot par l'unité de commande centrale par rapport à un point d'origine unique.

6. Système selon la revendication 3, ledit premier bras robotique (104) et ledit second bras robotique (105) étant positionnés par l'unité de commande centrale en position par rapport à l'anatomie osseuse du patient à l'aide de la caméra de navigation chirurgicale (107).

7. Système selon l'une quelconque des revendications précédentes, ladite unité de commande centrale étant en outre conçue pour détecter un contact électrique entre le premier fil rigide mince (113) et le second fil rigide mince (112).
